Europäisches Patentamt

European Patent Office

Office européen des brevets

(19)

(11) Publication number: **0 132 607**
**B1**

(12) **EUROPEAN PATENT SPECIFICATION**

(45) Date of publication of patent specification: **19.10.88**

(51) Int. Cl.⁴: **A 61 K 6/04**

(21) Application number: **84107349.7**

(22) Date of filing: **26.06.84**

(54) **Dental composition mixture.**

(30) Priority: **27.06.83 US 508368**

(43) Date of publication of application:
**13.02.85 Bulletin 85/07**

(45) Publication of the grant of the patent:
**19.10.88 Bulletin 88/42**

(84) Designated Contracting States:
**AT BE CH DE FR GB IT LI LU NL SE**

(56) References cited:
**EP-A-0 033 628**
**WO-A-83/02959**
**GB-A-1 580 132**
**GB-A-2 076 854**
**US-A-3 997 329**
**US-A-4 255 192**

(73) Proprietor: **DENTSPLY INTERNATIONAL, INC.**
**570 West College Avenue P.O. Box 872**
**York Pennsylvania 17405 (US)**

(72) Inventor: **Chodkowski, Michael R.**
**deceased (US)**

(74) Representative: **Wächtershäuser, Günter, Dr.**
**Tal 29**
**D-8000 München 2 (DE)**

The file contains technical information
submitted after the application was filed and
not included in this specification

Courier Press, Leamington Spa, England.

## Description

This invention relates to dental composition mixtures of cut and spherical alloys and especially to those containing silver, tin, copper, and titrated with mercury.

Dental alloy mixtures of said type have been described in US—A—4,255,192, GB—A—1,580,132, US—A—3,997,329, WO—A—8,302,959 and EP—A—0 033 628.

Dental amalgams are produced by intimately combining mercury with dental amalgam alloys. Conventional alloys have a major proportion of silver and a lesser proportion of tin and optionally contain other ingredients such as copper, zinc, and palladium. Upon reaction with mercury using known dental clinical techniques, a plastic mass is produced which quickly sets into a hard, rigid body. While the mass is plastic it may be packed into a surgically prepared tooth, restoring its anatomy and function.

The products of the amalgamation reaction are believed to be a silver-mercury reaction product ($Ag_2Hg_3$) and a tin-mercury reaction product ($Sn_{7-8}Hg$), referred to in the art as gamma-1 and gamma-2, respectively. It has been recognized that the presence of gamma-2 in dental amalgams is a source of corrosion in a saline environment. It is believed that the corrosion process probably releases mercury as a reaction product, resulting in the formation of additional voids and porosities. These may extend well below the surface since the gamma-2 phase in dental amalgam is interconnected. The excess mercury, voids, and porosities serve to weaken the dental amalgam especially at the margins which are the interfaces between the restoration and tooth. As a consequent of normal occlusion, stresses generated at a weakened margin may destroy its integrity, allowing leakage of oral fluids and bacteria, thereby promoting secondary decay.

Regardless of whether the aforementioned explanation of the corrosion process due to the presence of gamma-2 is correct (and the present invention is not necessarily limited thereto), it has been found that corrosion can be reduced by techniques which minimize, inhibit, or eliminate gamma-2 from dental amalgam compositions.

U.S. Patent 3,997,329 teaches the method of using a mixture of spheroid and irregularly shaped particles which when amalgamated with mercury exhibit enhanced dental properties. The aforesaid patent describes in some detail the characteristics of spheroidal particles and the characteristics of irregular shaped particles including the lathe-cut material which is particularly referred in the instant invention of the present application.

It is also known to use palladium and dental alloys to obtain the advantages thereof and an example of this is British Patent 2,076,854. In the past, a palladium containing alloy has been sold by the assignee of the present application. The alloy contained 49.5 plus or minus 5 percent silver (Ag), 30.0 plus or minus 5 percent tin (Sn), 20.0 plus or minus 5 percent copper (Cu), and 0.5 percent palladium (Pd).

## Objects of the invention

It is a general object of this invention to provide dental amalgam compositions which cope with the aforementioned problems of other amalgams.

It is a specific object to provide high-copper-content dental amalgamable alloys which are substantially free of mercury prior to amalgamation and which can be readily amalgamated without undue risks to personnel resulting from excessive mercury exposure.

It is another specific object to provide new dental amalgam compositions which upon amalgamation with mercury limit the gamma-2 phase which form and which provide enhanced physical, handling, and electrochemical properties.

It is another specific object to provide dental amalgam compositions which upon amalgamation with mercury substantially immediately inhibit gamma-2 formation so as to be substantially free of the gamma-2 phase and yet are competitive in cost with other amalgam compositions.

It is another specific object to provide dental amalgam compositions having improved properties upon amalgamation without unduly increasing the amounts of mercury required in the preparation thereof.

It is another specific object to provide a dental amalgam composition having condensing, working, carving, and adapting qualities superior to spheroidal amalgams or conventional irregularly-shaped microcut amalgams.

It is still another specific object to provide a dental amalgam which is substantially free of any tendency to ride up along walls of a cavity and packs firmly.

These and other objects will become apparent as the detailed description proceeds.

## Description of the invention

The present invention provides for a dental alloy mixture for use as a filling for dental preparations after amalgamation consisting essentially of:

(a) from 35% to 45% by weight of said mixture of cut alloy consisting essentially of 52% to 62% silver, 9% to 15% copper, 25% to 34% tin and 0% to 1% zinc by weight of said cut alloy; and

(b) from 55% to 65% by weight of said mixture of spherical alloy consisting essentially of 49% to 60% silver, 17% to 25% copper, 29% to 31% tin, and 0.2% to 1.2% palladium by weight of said spherical alloy.

The cut alloy is preferably composed of the following ingredients in the amounts indicated by weight:

| Element | Preferred | Most preferred |
|---|---|---|
| Silver | 52—62% | 54—60% |
| Tin | 34—25% | 32—27% |
| Copper | 15—12% | 14—13% |
| Zinc | 0—1% | 0 |

With the percentages being by weight of the total cut alloy composition.

A spherical alloy component is composed of the following elements in the proportions indicated by weight:

| Element | Preferred | Most preferred |
|---|---|---|
| Silver | 49—60% | 49.2—49.5% |
| Tin | 31—29% | 31—29% |
| Copper | 25—17% | 21—18% |
| Palladium | 0.2—1.2% | 0.3—1% |

The alloy mixture is preferably amalgamated with 40 to 55 percent mercury by weight of the combined weight of the mercury and alloy mixture, more preferably 45 to 50 percent mercury.

It is particularly important to maintain the copper content of the cut alloy between 9 and 15 percent and of a spherical alloy between 17 and 25 percent to provide sufficient copper content to react with the mercury released and limit the gamma 2 phase.

The Example

Fifty-seven grams of silver and 13.8 grams of copper was melted together at 1093°C in an induction furnace under an atmosphere of carbon monoxide. Twenty-nine and two tenths grams of tin were added to bring the melt temperature to 982°C. The liquid metal was poured into preheated cast iron ingot molds and then allowed to cool to ambient temperature. The resulting ingot slabs were annealed for 24 hours at 350°C under charcoal cover. A Horizontal Milling Machine cutter containing 22 teeth rotating at 170 rpm at a feed rate of 1.27 cm (0.5 inch) per minute was used to cut the ingots. The cut alloy was screened to 0.185 mm (80 mesh) and ball milled in a porcelain mill with porcelain grinding media for 60 minutes at 30 rpm. The resultant powder was screened to −0.044 mm (−325 mesh) and annealed at 100°C for 11 hours.

A suspension of 12.760 kg (450 ounces) of the annealed alloy in 4,500 ml of methanol was treated with hydrochloric acid, slurried and heated until the aqueous methanol was boiled away. The resultant powder was washed by decantation until the wash was at pH7, reslurried and treated with aqueous ammonium hydroxide. The ammonium hydroxide solution was decanted and the alloy washed with water until the wash solution was at pH7. The alloy was dried below 83°C and screened to −0.104 mm (−150 mesh). This alloy was referred to as the cut component.

The above product had the following composition: silver—57 percent, tin—29.2 percent, copper—13.8 percent which excluding any inherent impurities was 100 percent of the cut powder.

The spherical powder or component was a spherical alloy sold by The L. D. Caulk Company Division of Dentsply International Inc., the assignee of the present patent application, under the tradename Valiant, and had the following composition or assay: silver—49.5 percent, tin—30 percent, copper—20 percent, and palladium—0.5 percent. The cut powder and the spherical powder were combined in a ratio of 60 percent spherical to 40 percent cut by weight by tumble blending.

The mixed alloy composition was triturated with 47.7 percent mercury using a VARI-MIX® II M mechanical triturating device sold by The L. D. Caulk Division Dentsply International, at speed M2 for thirteen seconds with the following results:

3

| One-hour compressive strength | 208 MPa (30,100 PSI) |
| 24-hour compressive strength | 505 MPa (73,200 PSI) |
| 24-hour dimensional change | −6 µm/cm |
| 7-day static creep | 0.1 percent |
| (Eames) work time | 4 minutes |

The Eames test is set forth in a publication: Eames, W. B., and Skinner, EW 1965 International Association for Dental Research Program and Abstracts of Papers, Abstract 94, page 60.

The amalgam of Example I was used to provide a dental restoration and found to give excellent working characteristics and to perform well in the dental operatry. The dental preparation was made for receipt of the restorative amalgam material and the alloy composition was triturated with mercury and placed in a dental cavity preparation. The placed mass of alloy was packed into the dental preparation to substantially fill it and shaped in the conventional manner.

Example II

The above procedure was repeated except the compositions of the alloy powders were:

| Cut component | | Spherical component | |
| --- | --- | --- | --- |
| Silver | 59 | Silver | 49.5 |
| Tin | 29 | Tin | 30 |
| Copper | 11 | Copper | 20 |
| Zinc | 1 | Palladium | 0.5 |

60 percent spherical component by weight
40 percent cut component by weight

The mixed alloy composition was triturated with 49 percent of mercury in the manner of Example I and the results were:

| One-hour compressive strength | 210 MPa (30,400 PSI) |
| 24-hour compressive strength | 492 MPa (71,400 PSI) |
| 24-hour dimensional change | −3 µm/cm |
| 7-day static creep | 0.1 percent |
| (Eames) work time | 5 minutes |

Example III

The above procedure was repeated except the compositions of the alloy powders were:

| Cut alloy | | Spherical alloy | |
| --- | --- | --- | --- |
| Silver | 57.0 | Silver | 49.2 |
| Tin | 29.2 | Tin | 30 |
| Copper | 13.8 | Copper | 20 |
| Zinc | 0 | Palladium | 0.8 |

60 percent spherical component by weight
40 percent cut component by weight

The mixed alloy composition was triturated with 47.7 percent of mercury in the manner of Example I and the results were:

**0 132 607**

| | |
|---|---|
| One-hour compressive strength | 207 MPa (30,000 PSI) |
| 24-hour compressive strength | 457 MPa (66,200 PSI) |
| 24-hour dimensional change | −4 µm/cm |
| 7-day static creep | 0.1 percent |
| (Eames) work time | 4 minutes |

**Claims**

1. A dental alloy mixture for use as a filling for dental preparations after amalgamation consisting essentially of:

(a) from 35% to 45% by weight of said mixture of cut alloy consisting essentially of 52% to 62% silver, 9% to 15% copper, 25% to 34% tin and 0% to 1% zinc by weight of said cut alloy; and

(b) from 55% to 65% by weight of said mixture of spherical alloy consisting essentially of 49% to 60% silver, 17% to 25% copper, 29% to 31% tin, and 0.2% to 1.2% palladium by weight of said spherical alloy.

2. The dental alloy mixture of Claim 1 wherein said cut alloy is present in an amount between 35% and 40% of said mixture and said spherical alloy is present in an amount between 60% and 65% of said mixture.

3. The dental alloy mixture of Claim 1 wherein said cut component consists essentially of 54% to 60% silver 13%, to 14% copper, and 27% to 32% tin by weight of said cut alloy; and said spherical alloy consists essentially of 49.2% to 49.5% silver, 18% to 21% copper, 29% to 31% tin, and 0.3% to 1% palladium by weight of said spherical alloy.

4. The dental alloy mixture of Claim 1 amalgamated with 40% to 55% of mercury by weight of the combined weight of said mercury and said mixture.

5. The dental alloy mixture of Claim 3 triturated with 45% to 50% of mercury by weight of the combined weight of said mercury and said mixture.

**Patentansprüche**

1. Dentallegierungsmischung, die nach Amalgamierung als eine Füllung für dentale Präparationen verwendet wird, bestehend im wesentlichen aus:

(a) von 35 bis 45 Gew.-%, bezogen auf die Mischung, geschnittene Legierung, bestehend im wesentlichen aus 52 bis 62% Silber, 9 bis 15% Kupfer, 25 bis 34% Zinn und 0 bis 1% Zink, bezogen auf das Gewicht der geschnittenen Legierung; und

(b) von 55 bis 65 Gew.-%, bezogen auf die Mischung, sphärische Legierung, bestehend im wesentlichen aus 49 bis 60% Silber, 17 bis 25% Kupfer, 29 bis 31% Zinn und 0,2 bis 1,2% Palladium, bezogen auf das Gewicht der sphärischen Legierung.

2. Dentallegierungsmischung gemäß Anspruch 1, wobei die geschnittene Legierung in einer Menge zwischen 35% und 40% der Mischung vorliegt und die sphärische Legierung in einer Menge zwischen 60 und 65% der Mischung vorliegt.

3. Dentallegierungsmischung gemäß Anspruch 1, wobei die geschnittene Komponente im wesentlichen besteht aus 54 Bis 60% Silber, 13 bis 14% Kupfer und 27 bis 32% Zinn, bezogen auf das Gewicht der geschnittenen Legierung; und wobei die sphärische Legierung im wesentlichen besteht aus 49,2 bis 49,5% Silber, 18 bis 21% Kupfer, 29 bis 31% Zinn und 0,3 bis 1% Palladium, bezogen auf das Gewicht der sphärischen Legierung.

4. Dentallegierungsmischung gemäß Anspruch 1, amalgamiert mit 40 bis 55% Quecksilber, bezogen auf das kombinierte Gewicht des Quecksilbers und der Mischung.

5. Dentallegierungsmischung gemäß Anspruch 3, verrieben mit 45 bis 50 Gew.-% Quecksilber, bezogen auf das kombinierte Gewicht des Quecksilbers und der Mischung.

**Revendications**

1. Mélange d'alliage dentaire devant servir après amalgamation de charge pour préparations à usage dentaire composé essentiellement de:

(a) 35 à 45% en poids dudit mélange d'alliage fraisé composé essentiellement de 52 à 62% d'argent, 9 à 15% de cuivre, 25 à 34% d'étain et 0 à 1% de zinc en poids rapporté audit alliage fraisé; et

(b) 55 à 65% en poids dudit mélange d'alliage sphérique composé essentiellement de 49 à 60% d'argent, 17 à 25% de cuivre, 29 à 31% d'étain et 0,2% à 1,2% de palladium en poids rapporté audit alliage sphérique.

2. Mélange d'alliage dentaire selon la revendication 1 dans lequel ledit alliage fraisé est présent à raison de 35 à 40% dudit mélange et ledit alliage sphérique est présent à raison de 60 à 65% dudit mélange.

3. Mélange d'alliage dentaire selon la revendication 1 dans lequel ledit constituant fraisé est composé

5

essentiellement de 54 à 60% d'argent, 13 à 14% de cuivre et 27 à 32% d'étain en poids rapporté audit alliage fraisé; et ledit alliage sphérique est composé essentiellement de 49,2 à 49,5% d'argent, de 18 à 21% de cuivre, de 29 à 31% d'étain et de 0,3 à 1% de palladium en poids rapporté audit alliage sphérique.

4. Mélange d'alliage dentaire selon la revendication 1 amalgamé avec 40 à 55% de mercure en poids rapporté au poids total dudit mercure et dudit mélange.

5. Mélange d'alliage dentaire selon la revendication 3, trituré avec 45 à 50% de mercure en poids rapporté au poids total dudit mercure et dudit mélange.